(19) Europäisches Patentamt
European Patent Office
Office européen des brevets

(11) Veröffentlichungsnummer: **0 069 254**
**A1**

# (12) EUROPÄISCHE PATENTANMELDUNG

(21) Anmeldenummer: 82105265.1

(22) Anmeldetag: 16.06.82

(51) Int. Cl.³: C 07 D 487/04
A 61 K 31/415
//(C07D487/04, 235/00, 235/00)

(30) Priorität: 24.06.81 DE 3124701

(43) Veröffentlichungstag der Anmeldung: 12.01.83 Patentblatt 83/2

(84) Benannte Vertragsstaaten: AT BE CH DE FR GB IT LI LU NL SE

(71) Anmelder: BOEHRINGER INGELHEIM KG
ZA Patente
D-6507 Ingelheim am Rhein(DE)

(72) Erfinder: Stähle, Helmut, Dr.
Rotweinstrasse 23
D-6507 Ingelheim/Rhein(DE)

(72) Erfinder: Köppe, Herbert, Dr.
Neuweg 72
D-6507 Ingelheim/Rhein(DE)

(72) Erfinder: Kummer, Werner, Dr.
Georg-Scheuing-Strasse 15
D-6507 Ingelheim/Rhein(DE)

(72) Erfinder: Stockhaus, Klaus, Dr.
Pfarrer-Heberer-Strasse 35
D-6530 Bingen(DE)

(72) Erfinder: Hoefke, Wolfgang, Dr.
Rosselstrasse 21
D-6200 Wiesbaden(DE)

(72) Erfinder: Gaida, Wolfram, Dr.
Paul-Clemen-Strasse 14
D-6507 Ingelheim/Rhein(DE)

## (54) Neue 1-substituierte Imidazo(1,2-a)imidazole, deren Säureadditionssalze, diese enthaltende Arzneimittel und Verfahren zu deren Herstellung.

(57) Die Erfindung betrifft neue 1-substituierte 2-Methylen-1H-2,3,5,6-tetrahydro-imidazo[1,2-a]imidazole der allgemeinen Formel

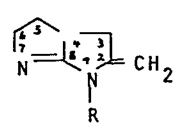

1

worin R einen ein- oder mehrfach durch ein Wasserstoff-, Fluor-, Chlor- oder Bromatom oder eine Methyl- oder Trifluormethylgruppe substituierten Phenylrest bedeutet sowie deren Säureadditionssalze.

Die neuen Verbindungen können durch Cyclisierung der Verbindungen der Formel II

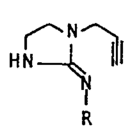

11

hergestellt werden und sind als Analgetika, Antihypertonika und Herz- und Coronartherapeutika verwendbar.

EP 0 069 254 A1

Croydon Printing Company Ltd.

COMPLETE DOCUMENT

Die Erfindung betrifft neue, in 1-Stellung substituierte 2-Methylen-1H-2,3,5,6-tetrahydro-imidazo[1,2-a]imidazole der allgemeinen Formel I

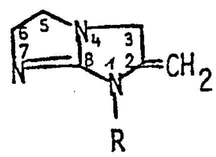

I

sowie deren physiologisch verträgliche Säureadditionssalze mit wertvollen therapeutischen Eigenschaften. In der Formel I bedeutet R einen ein- bis dreifach substituierten Phenylrest. Die Substituenten, welche gleich oder verschieden sein können, bedeuten ein Wasserstoff-, Fluor-, Chlor- oder Bromatom oder eine Methyl- oder Trifluormethylgruppe.

Die Herstellung der Imidazo[1,2-a]imidazole der allgemeinen Formel I erfolgt durch thermische Cyclisierung von Verbindungen der allgemeinen Formel II

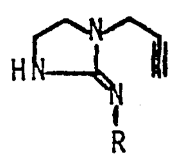

II

worin R wie oben angegeben definiert ist,bei Temperaturen zwischen 60 und 180°C. Die thermische Cyclisierung erfolgt zweckmäßigerweise durch Erhitzen der Verbindungen der allgemeinen Formel II in Gegenwart eines polaren oder unpolaren organischen Lösungsmittels auf Temperaturen von etwa 60 bis 180°C. Die speziellen Temperaturen hängen von der Reaktivität der jeweiligen Verbindungen der allgemeinen Formel II ab.

Neben den erfindungsgemäßen Verbindungen der allgemeinen

Formel I entstehen bei der thermischen Cyclisierung zusätzlich auch deren isomere Verbindungen der allgemeinen Formel III

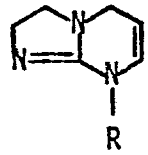

III
(R ist wie oben definiert)

die abgetrennt werden müssen.

Da sich die Isomeren der allgemeinen Formeln I und III in ihren pk-Werten deutlich voneinander unterscheiden ($pk_I < pk_{III}$), lassen sie sich aufgrund dieses Unterschiedes gut voneinander trennen. Durch fraktioniertes Extrahieren der wäßrigen Lösung eines Gemisches von Verbindungen der Formeln I und III bei aufsteigenden pH-Werten können die schwächer basischen erfindungsgemäßen Verbindungen der allgemeinen Formel I zuerst extrahiert und damit isoliert werden, während die stärker basischen Verbindungen der allgemeinen Formel III in der wäßrigen Lösung zurückbleiben.

Die Substanzen mit kleinerem pk-Wert, also die erfindungsgemäßen Verbindungen der allgemeinen Formel I, zeigen bei der Dünnschichtchromatografie (Kieselgel) in den Systemen
A= Toluol/Dioxan/Äthanol/konz. Ammoniak (50 : 40 : 5 : 5)
B= Essigester/Isopropanol/konz. Ammoniak (70 : 40 : 20)
C= sek.-Butanol/Ameisensäure (85 %)/$H_2O$ (75 : 15 : 10)
einen höheren $R_F$-Wert als die isomeren Verbindungen der allgemeinen Formel III.

Die Strukturen der neuen Imidazo[1,2-a]imidazole der allgemeinen Formel I sind durch $^1H$-bzw. $^{13}C$-Kernresonanz und massenspektroskopische Untersuchungen gesichert. Ausgangsverbindungen der allgemeinen Formel II sind bekannt und z.B. in der DE-OS 25 23 103 beschrieben.

Die erfindungsgemäßen Imidazo[1,2-a]imidazole der allgemeinen Formel I können auf übliche Weise in ihre physiologisch verträglichen Säureadditionssalze überführt werden. Zur Salzbildung geeignete Säuren sind beispielsweise Salzsäure, Bromwasserstoffsäure, Jodwasserstoffsäure, Fluorwasserstoffsäure, Schwefelsäure, Phosphorsäure, Salpetersäure, Essigsäure, Propionsäure, Buttersäure, Capronsäure, Valeriansäure, Oxalsäure, Malonsäure, Bernsteinsäure, Maleinsäure, Fumarsäure, Milchsäure, Weinsäure, Zitronensäure, Äpfelsäure, Benzoesäure, p-Hydroxybenzoesäure, p-Aminobenzoesäure, Phthalsäure, Zimtsäure, Salicylsäure, Ascorbinsäure, Methansulfonsäure, 8-Chlortheophyllin und dergleichen.

Die neuen Verbindungen der allgemeinen Formel I und deren Säureadditionssalze wirken analgetisch, blutdrucksenkend und herzfrequenzsenkend. Die analgetische Wirkung wurde nach dem Writhing-Test an der Maus geprüft. Darüber hinaus wurde bei der blutigen Messung des Blutdrucks am Kaninchen eine blutdrucksenkende Wirkung festgestellt. Die Beeinflussung der Herzfrequenz wurde an Spinalratten sowie an intakten narkotisierten Ratten untersucht. Aufgrund dieser Wirkungen kommen die Verbindungen der allgemeinen Formel I als Arzneimittel zur Behandlung von Schmerzzuständen, der Hypertonie und von Coronarerkrankungen in Frage. Die Wirkstoffe können enteral oder parenteral verabreicht werden. Die Dosierung liegt bei 0,1 bis 80, vorzugsweise 1 bis 30 mg.

Die Verbindungen der Formel I bzw. ihre Säureadditionssalze können auch mit andersartigen Wirkstoffen zum Einsatz gelangen. Geeignete galenische Darreichungsformen sind beispielsweise Tabletten, Kapseln, Zäpfchen, Lösungen oder

Pulver; hierbei können zu deren Herstellung die üblicherweise verwendeten galenischen Hilfs-, Träger-, Spreng- oder Schmiermittel oder Substanzen zur Erzielung einer Depotwirkung Anwendung finden.

Die folgenden Beispiele sollen die Erfindung und Herstellung der neuen Verbindungen erläutern,ohne sie zu beschränken.

Herstellungsbeispiele

Beispiel 1

1-(2,6-Dichlorphenyl)-2,3,5,6-tetrahydro-2-methylen-1H-imidazo[1,2-a]imidazol

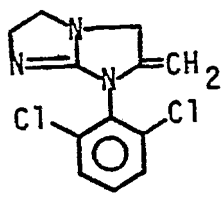

5,4 g 1-Propargyl-2-(2,6-dichlorphenylimino)-imidazolidin werden in 40 ml Äthanol 10 Stunden lang unter Rühren am Rückfluß erhitzt. Sodann wird die Reaktionsmischung im Vakuum zur Trockne eingedampft. Der Rückstand wird in 1 N HCl gelöst und die erhaltene Lösung bei aufsteigenden pH-Werten (Alkalisieren mit 2 N NaOH) fraktioniert mit Äther extrahiert. Das Ausgangsimidazolidin kann als erste Verbindung abgetrennt werden (Kontrolle durch Dünnschichtchromatogramm). Sobald es quantitativ entfernt ist, extrahiert man das neue Imidazo[1,2-a]imidazolderivat bei weiter aufsteigenden pH-Werten fraktioniert mit Äther (langsames stufenweises Alkalisieren mit 2 N NaOH; Dünnschichtchromatogramm-Kontrolle). Die dünnschichtchromatografisch-reinen Ätherextrakte werden vereinigt, über wasserfreiem Calciumsulfat getrocknet und der Äther im Vakkum abgezogen. Ausbeute: 0,85 g (15,9 % der Theorie). Schmelzpunkt: 165 - 168°C. Das Hydrobromid schmilzt bei 203 bis 204°C.

Beispiel 2

1-(2-Brom-6-fluor-phenyl)-2,3,5,6-tetrahydro-2-methylen-1H-imidazo[1,2-a]imidazol

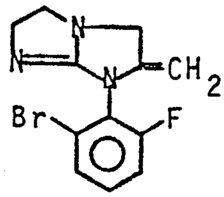

8,9 g 1-Propargyl-2-(2-brom-6-fluor-phenylimino)-imidazolidin werden in 60 ml absolutem Äthanol etwa 11 Stunden lang am Rückfluß erhitzt. Anschließend zieht man das Lösungsmittel im Vakuum ab und löst den verbleibenden Rückstand in verdünnter, etwa 1 N Salzsäure. Bei aufsteigenden pH-Werten (Alkalisieren mit 2 N NaOH) entfernt man sodann durch fraktionierte Extraktion mit Essigester das eingesetzte Imidazolidinderivat (Kontrolle durch Dünnschichtchromatogramm). Sobald das Ausgangsimidazolidin quantitativ entfernt ist, kann bei weiter aufsteigenden pH-Werten das erfindungsgemäße Imidazo[1,2-a]imidazolderivat extrahiert werden (vorsichtiges stufenweises Alkalisieren mit 2 N NaOH). Die einzelnen Extrakte werden nach Kontrolle durch Dünnschichtchromatogramm vereinigt, über wasserfreiem Calciumsulfat getrocknet und das Lösungsmittel im Vakuum entfernt.
Ausbeute: 0,6 g entsprechend 6,8 % der Theorie.
Schmelzpunkt: 122 bis 124°C.

Weitere Beispiele von Verbindungen der allgemeinen Formel I finden sich in der folgenden Tabelle.

| Beispiel Nr. | R | Ausbeute (% der Theorie) | Schmp. (°C) |
|---|---|---|---|
| 3 | 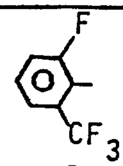 | 9,0 | 122-124 |
| 4 | 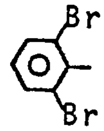 | 8,9 | 142-146 |
| 5 | 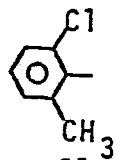 | 15,4 | 128-130 |
| 6 | 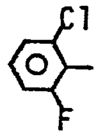 | 15,4 | 129-132 |
| 7 | 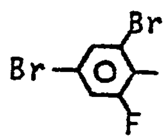 | 13,8 | 126-128 |
| 8 | 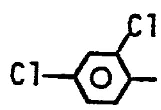 | 12,4 | 122-124 |
| 9 | 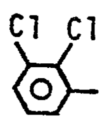 | 18,0 | 119-122 |
| 10 | 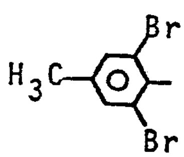 | 15,2 | 111-113 |

## Formulierungsbeispiele

### Beispiel A: Dragées

| | |
|---|---|
| Wirkstoff gemäß Erfindung | 5 mg |
| Milchzucker | 65 mg |
| Maisstärke | 130 mg |
| sek. Calciumphosphat | 40 mg |
| lösliche Stärke | 3 mg |
| Magnesiumstearat | 3 mg |
| kolloidale Kieselsäure | 4 mg |
| insgesamt | 250 mg |

Herstellung:

Der Wirkstoff wird mit einem Teil der Hilfsstoffe vermischt, intensiv mit einer wäßrigen Lösung der löslichen Stärke durchgeknetet und in üblicher Weise mit Hilfe eines Siebes granuliert. Das Granulat wird mit dem Rest der Hilfsstoffe vermischt und zu Drageekernen von 250 mg Gewicht verpreßt, die dann in üblicher Weise mit Hilfe von Zucker, Talkum und Gummi arabicum dragiert werden.

### Beispiel B: Ampullen

| | | |
|---|---|---|
| Wirkstoff gemäß Erfindung | | 1,0 mg |
| Natriumchlorid | | 18,0 mg |
| dest. Wasser | ad | 2,0 ml |

Herstellung:

Wirkstoff und Natriumchlorid werden in Wasser gelöst und unter Stickstoff in Glasampullen abgefüllt.

### Beispiel C: Tropfen

| | | |
|---|---|---|
| Wirkstoff gemäß Erfindung | | 0,02 g |
| p-Hydroxybenzoesäuremethylester | | 0,07 g |
| p-Hydroxybenzoesäurepropylester | | 0,03 g |
| entmineralisiertes Wasser | ad | 100 ml |

## P A T E N T A N S P R Ü C H E :

1. Neue 1-substituierte 2-Methylen-1H-2,3,5,6-tetrahydro-imidazo[1,2-a]imidazole der allgemeinen Formel

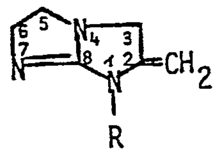

I

worin R einen ein- bis dreifach durch ein Wasserstoff-, Fluor-, Chlor- oder Bromatom oder eine Methyl- oder Trifluormethylgruppe substituierten Phenylrest, wobei die Substituenten gleich oder verschieden sein können, bedeutet sowie deren Säureadditionssalze.

2. Verfahren zur Herstellung von Verbindungen nach Anspruch 1, dadurch gekennzeichnet, daß man eine Verbindung der allgemeinen Formel

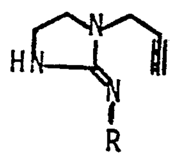

II

worin R wie oben angegeben definiert ist, bei Temperaturen von 60 bis 180°C cyclisiert und gegebenenfalls die erhaltene Base in ein Säureadditionssalz überführt.

3. Verfahren nach Anspruch 2, dadurch gekennzeichnet, daß man die Umsetzung in Gegenwart eines polaren oder unpolaren organischen Lösungsmittels durchführt.

4. Pharmazeutische Zubereitungen, dadurch gekennzeichnet, daß sie als Wirkstoff eine oder mehrere Verbindungen nach Anspruch 1 enthalten.

5. Verfahren zur Herstellung von Arzneimitteln nach Anspruch 4, dadurch gekennzeichnet, daß man eine oder mehrere Verbindungen nach Anspruch 1 mit üblichen galenischen Hilfs-, Träger-, Spreng- oder Schmiermitteln bzw. Substanzen zur Erzielung einer Depotwirkung zu Tabletten, Kapseln, Zäpfchen, Lösungen oder Pulver formuliert.

6. Verwendung von Verbindungen nach Anspruch 1 bei der Bekämpfung von Schmerzzuständen, Hypertonien und von Coronarerkrankungen.

0069254

Europäisches Patentamt

# EUROPÄISCHER RECHERCHENBERICHT

Nummer der Anmeldung

EP 82 10 5265

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
| A | EP-A-0 006 451 (BOEHRINGER)<br>* Ansprüche 1,4 *<br>----- | 1,4 | C 07 D 487/04<br>A 61 K 31/415//<br>(C 07 D 487/04<br>C 07 D 235/00<br>C 07 D 235/00 ) |
| | | | RECHERCHIERTE SACHGEBIETE (Int. Cl. 3) |
| | | | C 07 D 487/00<br>A 61 K 31/00 |

Der vorliegende Recherchenbericht wurde für alle Patentansprüche erstellt.

| Recherchenort | Abschlußdatum der Recherche | Prüfer |
|---|---|---|
| DEN HAAG | 01-10-1982 | ALFARO I. |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503. 03.82